# EUROPEAN PATENT APPLICATION

(11) **EP 4 234 538 A1**
(43) Date of publication of application: **30.08.2023**
(21) Application number: 20958439.0
(22) Date of filing: 02.11.2020
(51) Int. Cl.: C07C 323/59, C07C 229/08, C07C 229/24, C07C 279/14

(54) **REFINING METHOD FOR AMINO ACID OR DERIVATIVES THEREOF, AND AMINO ACID OR DERIVATIVES THEREOF**

(30) Priority: 22.10.2020 WO PCT/CN2020/122899
(71) Applicant: Wuhan Grand Hoyo Co., Ltd., Wuhan, Hubei 430074 (CN)
(72) Inventor: ZHU, Chengjun, Wuhan, Hubei 430074 (CN); SHU, Min, Wuhan, Hubei 430074 (CN); LI, Yun, Wuhan, Hubei 430074 (CN); HUANG, Lei, Wuhan, Hubei 430074 (CN); TANG, Peng, Wuhan, Hubei 430074 (CN); LI, Jianhua, Wuhan, Hubei 430074 (CN); SU, Haixia, Wuhan, Hubei 430074 (CN); ZHA, Liyan, Wuhan, Hubei 430074 (CN); GUO, Liping, Wuhan, Hubei 430074 (CN); LIU, Mengjie, Wuhan, Hubei 430074 (CN); HE, Jiajun, Wuhan, Hubei 430074 (CN); YANG, Lei, Wuhan, Hubei 430074 (CN); LIU, Sha, Wuhan, Hubei 430074 (CN); LV, Fanlin, Wuhan, Hubei 430074 (CN); MEI, Xuechen, Wuhan, Hubei 430074 (CN); LIU, Zhen, Wuhan, Hubei 430074 (CN)
(74) Representative: Wilson, Justin Scott
(86) International application number: PCT/CN2020/125919
(87) International publication number: WO 2022/082859

(57) **Abstract**

Provided is a refining method of an amino acid or derivatives thereof, including: (1) preparing a saturated aqueous solution of the amino acid or derivatives thereof by using a crude product of the amino acid or derivatives thereof at a preset temperature; (2) cooling, in a first programmed cooling, the saturated aqueous solution to a metastable zone, adding crystal seeds having a sphericity degree not less than 0.6, and performing constant-temperature crystal cultivating to obtain a crystal slurry; and (3) cooling, in a second programmed cooling, the crystal slurry to a second temperature with a cooling rate not higher than 1.0°C/min, and performing constant-temperature crystal cultivating to obtain the amino acid or derivatives thereof.

## Description

### CROSS-REFERENCE TO RELATED APPLICATIONS

This application claims priority to and the benefits of Patent Application No. PCT/CN2020/122899, filed to the China National Intellectual Property Administration on October 22, 2020, the entire disclosure of which is incorporated herein by reference.

### FIELD

The present disclosure relates to the technical field of medicine, and particularly, to a refining method of amino acid or derivatives thereof, and the amino acid or derivatives thereof.

### BACKGROUND

Drug polymorphism is one of the important factors affecting drug quality and clinical efficacy. In drug quality control, the crystal form is one of the important quality control indicators. Drugs with different crystal forms have different properties. These properties will not only affect the fluidity, compressibility, cohesiveness, and other processing properties of pharmaceutical products, and more importantly, they may cause quality differences in drug dissolution rate, dissolution degree, stability, etc., thereby affecting the biological activity and bioavailability of drugs, and leading to differences in clinical efficacy.

In the pharmaceutical industry, the fluidity of crystals is of great significance to the production, mixing of different components, transport, storage, and other processes of products. If the crystal fluidity is poor, the various components can be hardly mixed in the drug evenly, resulting in large differences in the drug. In the process of transportation and storage of preparations, crystals with poor fluidity may reduce the stability and effectiveness of drugs due to the influence of external environmental temperature, humidity, pressure, and other factors. At present, an angle of repose is adopted to characterize the fluidity of powders: when the angle of repose is less than 30°, the fluidity is excellent; when the angle of repose is 30° to 45°, the fluidity is good; and when the angle of repose is greater than 45°, the liquidity is poor.

Acetylcysteine, with chemical name of N-acetyl-L-cysteine, also called Tixair, Fluimucil, Broncholysin, Airbron, etc., is a precursor of reduced glutathione (GSH) and a typical amino acid, with a molecular formula C₅H₉NO₃S and a molecular weight of 162.19. The product is a white crystalline powder with a melting point of 101°C to 107°C, and has an odor like garlic, a sour taste, and hygroscopicity. Acetylcysteine is easily soluble in water and ethanol and insoluble in dichloromethane and ether, and has the following structure:

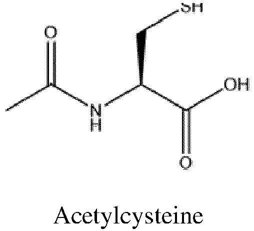

Chinese patent CN 104844488 B discloses an acetylcysteine refining process, which includes dissolving a crude product with water, directly cooling and crystallizing, centrifuging, collecting crystals, and drying, to obtain pure acetylcysteine. However, the product obtained by this process is slender and pointed and has poor fluidity, prone to be broken, and the product has uneven particle size distribution, many fine crystals, agglomeration in the drying process, and poor product quality, which does not meet the requirements of high-end pharmaceutical drugs.

At present, the preparation processes of amino acids or derivatives thereof on the market are relatively rough, and the prepared amino acids or derivatives thereof are slender and pointed, have poor liquidity, and they are prone to be broken. With the increasingly strict supervision of drug quality and the increasing requirements of high-end pharmaceutical market, it is necessary to develop a preparation process that can effectively improve the quality of amino acids or derivatives thereof.

### SUMMARY

The present disclosure provides a refining method, which can effectively improve the product quality of amino acids or derivatives thereof.

Another object of the present disclosure is to provide amino acids or derivatives thereof with high sphericity degree and good fluidity.

In order to achieve the first purpose of the present disclosure, the present disclosure provides a refining method of an amino acid or derivatives thereof, including: (1) preparing a saturated aqueous solution of the amino acid or derivatives thereof by using a crude product of the amino acid or derivatives thereof at a preset temperature; (2) cooling, in a first programmed cooling, the saturated aqueous solution to a metastable zone, adding crystal seeds having a sphericity degree not less than 0.6, and performing constant-temperature crystal cultivating to obtain a crystal slurry; and (3) cooling, in a second programmed cooling, the crystal slurry to a second temperature with a cooling rate not higher than 1.0°C/min, and performing constant-temperature crystal cultivating to obtain the amino acid or derivatives thereof.

The Applicant found in researches that, for amino acids or derivatives thereof, the crystal habit and particle size of drugs also have direct influences on the quality control of the drugs in addition to the crystal form of drugs. For active pharmaceutical ingredients, long rod-shaped and needle-shaped crystals have many plane contact points on the surfaces thereof and shear forces between irregular particles, and thus they have poor fluidity. During the tableting process, the particles of the crystals may be evenly filled in the mold hole and prone to be broken and prone to agglomerate, thereby affecting the quality of the later tableting products. In contrast, spherical particles have the smallest contact area with each other and the optimal fluidity, such that they are suitable for direct tableting, and they also have high crystal stability, which can effectively improve the quality of products.

On the other hand, if the crystals have uneven and small particle sizes, they are likely to agglomerate and have poor fluidity, which is not conducive to the tableting and manufacturing process. In addition, impurities may be easily incorporated, which has a significant impact on the product quality. Specifically, when the particle size distribution is uneven, the particle flow rate varies during tableting, and the particles filled in the mold hole therefore have uneven sizes or have a significant difference in particle size. Thus, large particles are separated from small particles due to the vibration of equipment during feeding, causing that the difference in tablet weight exceeds the limit during filling.

According to the embodiments of the present disclosure, the method can improve the sphericity degree and particle size uniformity of amino acids or derivative products thereof. In this way, the prepared amino acids or derivative products thereof have excellent fluidity performance and high crystal stability, and they are suitable for direct tableting, thereby effectively improving the quality of tablet products.

According to the embodiment of the present disclosure, the above method can further include at least one of the following additional technical features.

In the method according to an embodiment of the present disclosure, the crude product is dissolved in the solvent rapidly through heating, and thus spherical crystals can also be easily formed in the subsequent two-step cooling. The Applicant found that the method of the present disclosure is suitable for refining a variety of substances, especially for refining amino acids and related derivatives. The obtained crystals have high sphericity degree and uniform particle size and are not prone to aggregate.

According to an embodiment of the present disclosure, the solvent is water. According to the method of the embodiment of the present disclosure, the raw material of the solvent is easily available, which is convenient for industrial production. In addition, when water is used as the solvent, the organic residue can be effectively avoided when alcohol substances (such as ethanol) are used as the solvent. Moreover, when water is used as the solvent, the crystals can have a higher sphericity degree, thereby avoiding the pointed and slender crystal habit when using the alcohol solvent.

According to an embodiment of the present disclosure, the preset temperature ranges from 40°C to 80°C, preferably from 50°C to 80°C. According to the method of the embodiment of the present disclosure, the preset temperature is 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, or 80°C. At the above-mentioned temperature, the crude product has a high solubility during dissolving, and the dissolution of the crude product can be advantageously accelerated, thereby ensuring complete dissolution. Moreover, the preset temperature being the above temperature is conducive to the subsequent two-step cooling and the subsequent forming of crystals with high sphericity degree.

According to an embodiment of the present disclosure, said cooling, in the first programmed cooling, the solution to be crystallized to the metastable zone is carried out under stirring. Optionally, a speed of the stirring ranges from 100 to 600 r/min, and preferably from 300 to 500 r/min. Specifically, the speed of the stirring is 100 r/min, 200 r/min, 300 r/min, 400 r/min, 500 r/min, or 600 r/min. The Applicant found that the above-mentioned speed of the stirring is appropriate and can avoid the incomplete crystal habit, occurrence of fine grains, uneven particle size distribution, and crystal agglomeration, which may occur when the speed of the stirring is excessively high, and the above-mentioned speed of the stirring can also avoid the phenomenon of local oversaturation, crystal deposition and agglomeration, and uneven particle size distribution, which may occur when the speed of the stirring is excessively small.

According to an embodiment of the present disclosure, the metastable zone is between 25°C and 50°C, and preferably between 27°C to 45°C. According to the method of the embodiment of the present disclosure, the metastable zone is at 25°C, 26°C, 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, 45°C, 46°C, 47°C, 48°C, 49°C, or 50°C. At the above-mentioned temperatures are each a temperature at which the crystals can reach the supersaturated state, that is, the crystals are in the metastable zone but have not been precipitated. When the crystal seeds with a sphericity degree not less than 0.6 are added at the above-mentioned temperature, the finally obtained crystal can have a high sphericity degree and uniform particle size distribution. However, when the long-pointed seeds with a low sphericity degree are added, the obtained crystals have a low sphericity degree and a long rod-shaped crystal habit.

The purpose of cooling the saturated solution of the amino acid or derivatives thereof from a higher temperature to the metastable zone is to allow the solution to be in a supersaturated state and to generate a driving force for the crystal cultivating process without crystal precipitation, which is conducive to the subsequent operation of adding crystal seeds. Those skilled in the art can understand that a zone between the solubility equilibrium curve and the supersolubility curve is the metastable zone for crystal cultivating, and solutions of different amino acids or derivatives thereof have different temperature range of the metastable zone, which is specifically related to their physical properties and solubility. Similarly, even for the same amino acid or derivatives thereof, its metastable zone also has a certain dynamic change law and is closely related to the stirring rate and cooling rate. However, those skilled in the art can understand that, for any specific amino acid or derivatives thereof, as long as the conditions for refining are fixed, the metastable zone of the solution of this amino acid or derivatives thereof is definite, and the purpose of cooling the saturated solution of the amino acid or derivatives thereof from a higher temperature to the metastable zone is also definite and achievable.

According to an embodiment of the present disclosure, the cooling rate of the first programmed cooling ranges from 1.0°C/min to 6.0°C/min, preferably from 1.0°C/min to 3.0°C/min. According to the method of the embodiment of the present disclosure, the cooling rate of the first programmed cooling is 1°C/min, 1.5°C/min, 2°C/min, 2.5°C/min, 3°C/min, 3.5°C/min, 4°C/min, 4.5°C/min, 5°C/min, 5.5°C/min, or 6°C/min. Under the high cooling rate, the metastable zone is wider, and the crystal cultivating solution can quickly reach the metastable zone, and the operable zone of the crystal cultivating temperature is thus wider, facilitating the precipitation of subsequent spherical crystals, saving the cooling time, shortening the entire refining time, and improving the production efficiency.

According to an embodiment of the present disclosure, the sphericity degree of the crystal seeds preferably ranges from 0.6 to 0.9, the crystal seeds are added in an amount of 1% to 15% by mass, based on the saturated aqueous solution, and the crystal seeds have a main particle size ranging from 10 µm to 200 µm. Preferably, the crystal seeds are added in an amount of 2% to 6% by mass, and the crystal seeds have a main particle size ranging from 50 µm to 125 µm. According to the method of the embodiment of the present disclosure, when the seeds are added in a relatively higher amount and the seeds have a greater main particle size and a higher sphericity degree, the prepared crystals can have a uniform particle size and high stability, and they are not prone to coalesce, which is conducive to the generation of crystals with a high sphericity degree.

According to the embodiment of the present disclosure, a crystal habit sphericity degree of the crude product of the amino acid or derivatives thereof is not higher than 0.5.

According to the embodiment of the present disclosure, the constant-temperature crystal cultivating lasts for 5 to 60 min, preferably 20 to 40 min. The crystal cultivating time according to the embodiment of the present disclosure provides sufficient aging time to the crystals, which is conducive to crystal precipitation and crystal integrity. The obtained crystal particle size is large and uniform, which is conducive to subsequent filtering, reducing filtering time and improving the production efficiency.

According to an embodiment of the present disclosure, the second temperature ranges from 0°C to 25°C, preferably from 5°C to 15°C. According to the method of the embodiment of the present disclosure, the second temperature is 0°C, 1°C, 2°C, 3°C, 4°C, 5°C, 6°C, 7°C, 8°C, 9°C, 10°C, 11°C, 12°C, 13°C, 14°C, 15°C, 16°C, 17°C, 18°C, 19°C, 20°C, 21°C, 22°C, 23°C, 24°C, or 25°C. The second temperature is lower than the temperature of the metastable zone, and the second temperature is a low temperature, which is conducive to the growth of crystals and the increase of yield.

According to an embodiment of the present disclosure, the cooling rate of the second programmed cooling is not higher than 1.0°C/min, preferably between 0.2°C/min and 1.0°C/min. According to the method of the embodiment of the present disclosure, the cooling rate of the second programmed cooling is 0.1°C/min, 0.2°C/min, 0.3°C/min, 0.4°C/min, 0.5°C/min, 0.6°C/min, 0.7°C/min, 0.8°C/min, 0.9°C/min, or 1.0°C/min. The second cooling rate is suitable to allow the crystal slurry to slowly reach the second temperature, which is conducive to crystal precipitation and crystal growth, thereby avoiding problems such as uneven product particle size and inclusion of impurities due to too fast cooling.

According to an embodiment of the present disclosure, after said cooling the crystal slurry in the second programmed cooling and said performing the constant-temperature crystal cultivating, the method further includes: performing centrifugal filtration and drying on the product. According to an embodiment of the present disclosure, a rotating speed of the centrifugal filtering ranges from 1,000 rpm to 5,000 rpm, and preferably, from 3,000 rpm to 4,500 rpm. According to the method of the embodiment of the present disclosure, the rotation speed cannot damage the crystals and can allow the crystals to be filtered quickly, so that the wet product has a small moisture content, avoiding product caking due to a high moisture content in the subsequent drying process.

According to an embodiment of the present disclosure, the drying includes at least one of 75 to 80°C atmospheric drying, vacuum drying, or rotary evaporation drying.

According to the embodiment of the present disclosure, the amino acid or derivatives thereof prepared by the method of the present disclosure has a crystal habit sphericity degree not less than 0.8. The crystals prepared by the method according to the embodiment of the present disclosure have a high crystal habit sphericity degree and good fluidity, and thus they are suitable for tableting.

According to an embodiment of the present disclosure, the amino acid or derivatives thereof prepared by the method of the present disclosure has a span of particle size distribution not higher than 1.1. When the crystals prepared by the method according to the embodiment of the present disclosure have the span of particle size distribution not higher than 1.1, the crystals have a high stability, which can improve the quality of tablets.

In order to realize a second purpose of the present disclosure, the present disclosure provides an amino acid or derivatives thereof. The amino acid or derivatives thereof is characterized in that the refined product of the amino acid or derivatives thereof has a crystal habit sphericity degree not less than 0.8 and a span of particle size distribution not higher than 1.1. According to the embodiment of the present disclosure, the amino acid or derivatives thereof have excellent fluidity performance and have high crystal stability, and they, are suitable for direct tableting, thereby effectively improving the quality of tablet products. The Applicant found that, for the active pharmaceutical ingredients, long rod-shaped and needle-shaped crystal raw materials have many plane contact points on the surfaces thereof and shear forces between irregular particles, and thus they have poor fluidity. During the tableting process, the particles of the crystals may be evenly filled in the mold hole and prone to be broken and prone to agglomerate, thereby affecting the quality of the later tableting products. During the forming or filling of capsules, tablets, granules, etc., due to the poor fluidity and rough surfaces of the crystals, they are prone to agglomerate, causing inconvenience for patients' oral administration and increasing the cost of the manufacturing process. In contrast, spherical particles have the smallest contact area with each other and the optimal fluidity, such that they are suitable for direct tableting, and they also have high crystal stability, which can effectively improve the quality of products.

According to the embodiment of the present disclosure, the above-mentioned amino acid or derivatives thereof may further include at least one of the following additional technical features.

According to an embodiment of the present disclosure, the amino acid or derivatives thereof includes at least one of acetylcysteine, alanine, arginine, glycine, proline, hydroxyproline, serine, ornithine hydrochloride, arginine hydrochloride, lysine hydrochloride, or histidine hydrochloride. Preferably, the amino acid or derivatives thereof includes acetylcysteine, ornithine hydrochloride, glycine, or alanine. More preferably, the amino acid is acetylcysteine. In the process of research and development, the Applicant found that, for other kinds of amino acids having similar solubility in water as acetylcysteine, the refining method provided by the present disclosure can also obtain higher spherical crystal habit and particle size distribution results, with good effects. This method is more advantageous and has higher refining efficiency in refining acetylcysteine.

In order to achieve a third purpose of the present disclosure, the present disclosure provides a method for refining acetylcysteine, including: preparing a saturated aqueous solution of acetylcysteine from an acetylcysteine crude product at 50 to 80°C; cooling the saturated aqueous solution to a metastable zone between 27 °C and 45°C at a rate of 1°C/min to 3.0°C/min, adding crystal seeds having a sphericity degree not less than 0.6, and performing constant-temperature crystal cultivating to obtain a crystal slurry; and cooling the crystal slurry to 5 to 15°C at a rate of 0.2°C/min to 0.8°C/min, and performing constant-temperature crystal cultivating to obtain acetylcysteine. According to an embodiment of the present disclosure, before the crystal seeds are added, it is necessary to performing at a rate of 1°C/min to 3.0°C/min to reach a temperature of 27°C, 28°C, 29°C, 30°C, 31°C, 32°C, 33°C, 34°C, 35°C, 36°C, 37°C, 38°C, 39°C, 40°C, 41°C, 42°C, 43°C, 44°C, or 45°C. Such a temperature is a temperature at which acetylcysteine crystals reach a supersaturation state, that is, acetylcysteine crystals are in the acetylcysteine metastable zone but have not been precipitated. At such a temperature, by adding crystal seeds with a sphericity degree not less than 0.6 to grow the crystals, the finally obtained crystals can have a high sphericity degree and uniform particle size distribution.

Additional aspects and advantages of the present disclosure will be given in part in the following description, and in part will become apparent from the following description, or learned from the practice of the present disclosure.

### BRIEF DESCRIPTION OF THE DRAWINGS

The above and/or additional aspects and advantages of the present disclosure will become apparent and easily understood from the description of embodiments in conjunction with the following drawings, wherein:
FIG. 1 is an X-ray diffraction (XRD) comparison diagram of a raw material and acetylcysteine prepared according to Example 1 to Example 5 and Comparative Example 1 to Comparative Example 3 of the present disclosure;
FIG. 2 is an XRD comparison diagram of a raw material and ornithine hydrochloride prepared according to Example 6 and Comparative Example 4 of the present disclosure;
FIG. 3 is a polarized microscope photograph of acetylcysteine prepared according to Example 1 of the present disclosure;
FIG. 4 is a particle size distribution of acetylcysteine prepared according to Example 1 of the present disclosure;
FIG. 5 is a polarized microscope photograph of acetylcysteine prepared according to Example 2 of the present disclosure;
FIG. 6 is a particle size distribution of acetylcysteine prepared according to Example 2 of the present disclosure;
FIG. 7 is a polarized microscope photograph of acetylcysteine prepared according to Example 3 of the present disclosure;
FIG. 8 is the particle size distribution of acetylcysteine prepared according to Example 3 of the present disclosure;
FIG. 9 is a polarized microscope photograph of acetylcysteine prepared according to Example 4 of the present disclosure;
FIG. 10 is a particle size distribution of acetylcysteine prepared according to Example 4 of the present disclosure;
FIG. 11 is a polarized microscope photograph of acetylcysteine prepared according to Example 5 of the present disclosure;
FIG. 12 is a particle size distribution of acetylcysteine prepared according to Example 5 of the present disclosure;
FIG. 13 is a polarized microscope photograph of ornithine hydrochloride prepared according to Example 6 of the present disclosure;
FIG. 14 is a particle size distribution of ornithine hydrochloride prepared according to Example 6 of the present disclosure;
FIG. 15 is a polarized microscope photograph of glycine prepared according to Example 7 of the present disclosure;
FIG. 16 is a particle size distribution of glycine prepared according to Example 7 of the present disclosure;
FIG. 17 is a polarized microscope photograph of alanine prepared according to Example 8 of the present disclosure;
FIG. 18 is a particle size distribution of alanine prepared according to Example 8 of the present disclosure;
FIG. 19 is a polarized microscope photograph of acetylcysteine prepared according to Comparative Example 1 of the present disclosure;
FIG. 20 is a particle size distribution of acetylcysteine prepared according to Comparative Example 1 of the present disclosure;
FIG. 21 is a polarized microscope photograph of acetylcysteine prepared according to Comparative Example 2 of the present disclosure;
FIG. 22 is a particle size distribution of acetylcysteine prepared according to Comparative Example 2 of the present disclosure;
FIG. 23 is a polarized microscope photograph of acetylcysteine prepared according to Comparative Example 3 of the present disclosure;
FIG. 24 is a particle size distribution of acetylcysteine prepared according to Comparative Example 3 of the present disclosure;
FIG. 25 is a polarized microscope photograph of ornithine hydrochloride prepared according to Comparative Example 4 of the present disclosure; and
FIG. 26 is a particle size distribution of ornithine hydrochloride prepared according to Comparative Example 4 of the present disclosure.

### DETAILED DESCRIPTION

The embodiments of the present disclosure are described in detail below, and the examples of the embodiments are illustrated in the drawings. The embodiments described below with reference to the accompanying drawings are exemplary and are intended to explain the present disclosure, but cannot be construed as a limitation of the present disclosure.

### Explanation of terms

Unless otherwise specified, "first", "second", "third", and other similar terms used herein are for the purpose of convenient description and distinguishing, and they neither imply or express differences in order or importance for any purpose, nor indicate that the content defined by "first", "second", "third", or other similar terms is composed of only one component.

The solutions according to the present disclosure will be explained by means of examples. Those skilled in the art can understand that the following examples are only used to explain the present disclosure, rather than limiting the scope of the present disclosure. The reagents or instruments used in the examples without indicating the manufacturers shall be the conventional and commercially available products, for example, they can be purchased from Illumina.

The instruments used in the examples and comparative examples of the present disclosure are illustrated in Table 1.

**Table 1: Instrument Information**

| Instrument Name | Manufacturer | Mode |
|---|---|---|
| Jacketed crystallizer | Zhengzhou Jinshui District Xinrui Glassware Business Department | 500 mL |
| Digital display mixer | Shanghai Sile Instrument Co., Ltd | HD2010W |
| Uniform speed programmed thermostatic bath | Nanjing Xianou Instrument Manufacturing Co., Ltd | XOYS-2006N |
| Centrifuge | Shanghai Lu Xiangyi Centrifuge Instrument Co., Ltd | TD5GL |
| X-ray powder diffractometer | Bruker | D8 advance |
| Polarizing microscope | Leica | DM750P |
| Laser particle size analyzer | Malvern | MS3000 |

### Example 1

200.00 g of an acetylcysteine crude product was dissolved in 100.0 mL of water at a preset temperature of 75°C under stirring at a rate of 300 r/min to prepare a saturated solution of acetylcysteine. Then, the solution was cooled to 45°C at a rate of 1.0°C/min, and added with 5.28 g of ellipsoidal crystal seeds having a main particle size of 50 µm and a sphericity degree of 0.783, followed by constant-temperature crystal cultivating for 40 min, to obtain a crystal slurry. Then, the crystal slurry was cooled to 10°C at a cooling rate of 0.3°C/min, followed by crystal cultivating for 30 min, centrifugation, filtration, and drying, to obtain 173.98 g of acetylcysteine crystals.

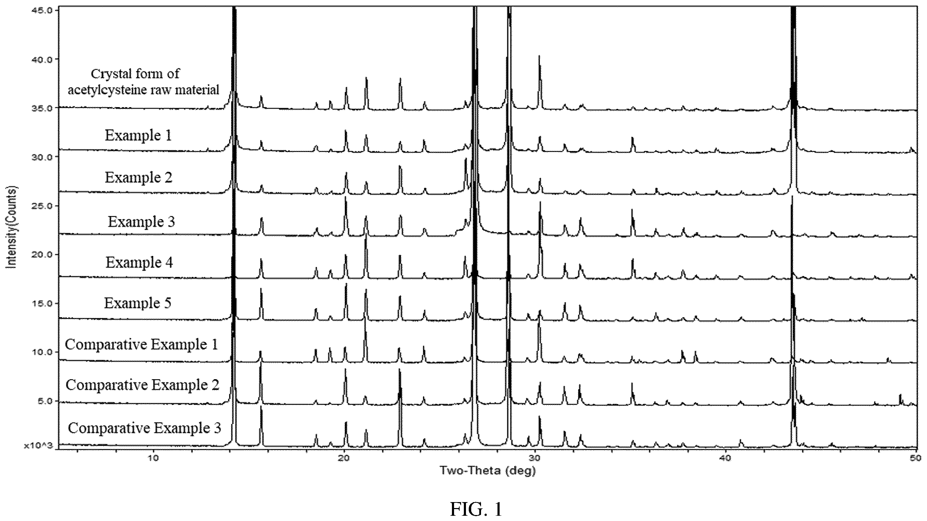

The XRD spectrum of the acetylcysteine product obtained in this example is illustrated in FIG. 1. Compared with the crystal forms disclosed in the literature (CrysEngComm, 2013, 15:6498-6505), the peak position of the product obtained by the new crystallization process in Example 1 is consistent with that of crystal form I, indicating that the acetylcysteine product obtained by the new crystallization process is pure crystal form I.

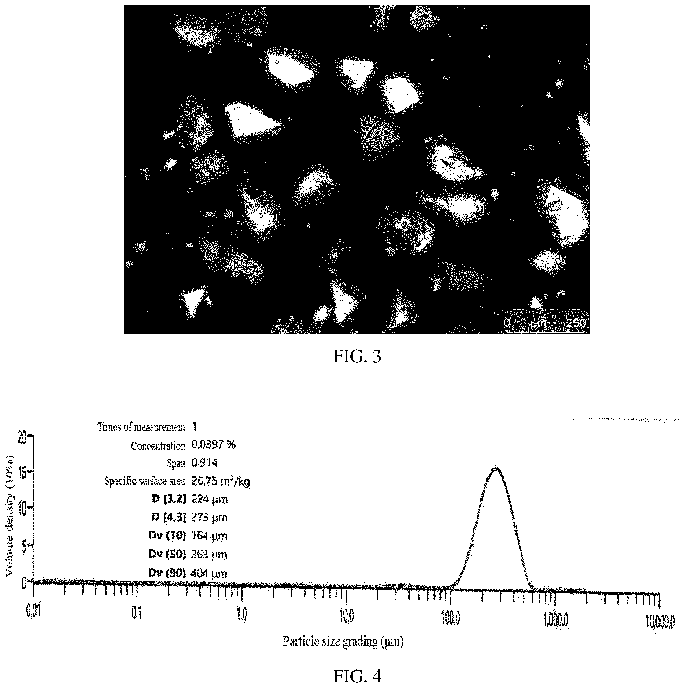

The microscope photograph of the acetylcysteine product prepared in this example illustrated in FIG. 3. FIG. 3 reveals that the crystal habit of the prepared acetylcysteine is ellipsoidal crystal with a sphericity degree of 0.865, the crystal habit is complete, and no agglomeration exits.

The particle size of the product obtained in this example was determined by Mastersizer 3000 laser particle size analyzer. The particle size distribution is illustrated in FIG. 4. The particle size distribution is unimodal and has a span of 0.914. The particle size distribution is relatively concentrated, and the mean particle size D[4,3] is 273 µm.

The product obtained in this example has an angle of repose of 28°, which was measured by the fixed funnel method, indicating excellent fluidity.

### Example 2

150.00 g of an acetylcysteine crude product was dissolved in 100.0 mL of water preset at a temperature of 60°C under stirring at a rate of 350 r/min to prepare a saturated solution of acetylcysteine. Then, the solution was cooled to 43°C at a rate of 1.25°C/min, and added with 3.48 g of ellipsoidal crystal seeds having a main particle size of 80 µm and a sphericity degree of 0.803, followed by constant-temperature crystal cultivating for 30 min, to obtain a crystal slurry. The crystal slurry was cooled to 5°C at a cooling rate of 0.5°C/min, followed by crystal cultivating for 30 min, vacuum filtration, and drying, to obtain 131.85g of acetylcysteine crystals.

The XRD spectrum of the acetylcysteine product prepared in this example is illustrated in FIG. 1. Compared with the two crystal forms disclosed in the literature (CrystEngComm, 2013, 15:6498-6505), the peak position of the crystal product obtained by the new crystallization process in Example 2 is consistent with that of crystal form I, indicating that the acetylcysteine product obtained by the new crystallization process is pure crystal form I.

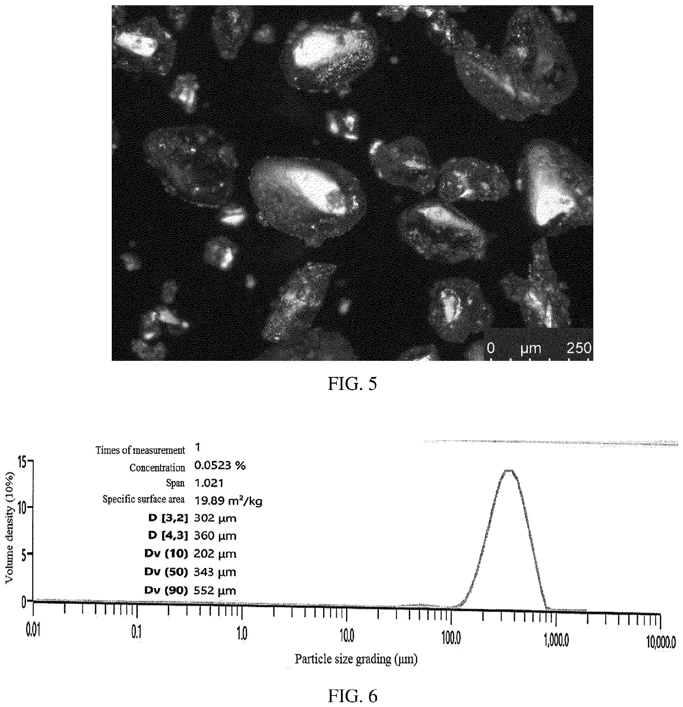

The microscope photograph of the acetylcysteine product prepared in this example is illustrated in FIG. 5. FIG. 5 reveals that the crystal habit of the prepared acetylcysteine is ellipsoidal crystal with a sphericity degree of 0.881, the crystal habit is complete, and no agglomeration exits.

The particle size of the product obtained in this example was determined by Mastersizer 3000 laser particle size analyzer. The particle size distribution is illustrated in FIG. 6. The particle size distribution is unimodal and has a span of 1.021. The particle size distribution is relatively concentrated, and the mean particle size D[4,3] is 360 µm.

The product obtained in this example has an angle of repose of 26°, which was measured by the fixed funnel method, indicating excellent fluidity.

### Example 3

100.00 g of an acetylcysteine crude product was stirred and dissolved in 100.0 mL of water at a preset temperature of 55°C under stirring at a rate of 400 r/min to prepare a saturated solution of acetylcysteine. Then, the solution was cooled to 30°C at a rate of 2.0°C/min, and added with 3.01 g of ellipsoidal crystal seeds having a main particle size of 100 µm and a sphericity degree of 0.794, followed by constant-temperature crystal cultivating for 40 min. Thereafter, the obtained solution was cooled to 5°C at a cooling rate of 0.8 °C/min, followed by crystal cultivating for 25min, vacuum filtration, and drying, to obtain 86.64 g of acetylcysteine crystals.

The XRD spectrum of the acetylcysteine product prepared in this example is illustrated in FIG. 1. Compared with the two crystal forms in the literature (CrystEngComm, 2013, 15:6498-6505), the peak position of the crystal product obtained by the new crystallization process in Example 3 is consistent with that of crystal form I, indicating that the acetylcysteine product obtained by the new crystallization process is pure crystal form I.

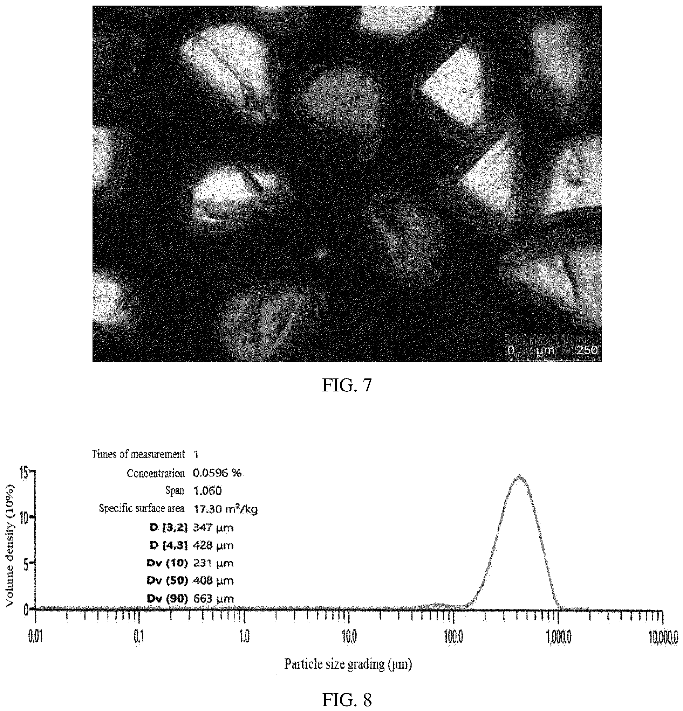

The microscope photograph of the acetylcysteine product prepared in this example is illustrated in FIG. 7. FIG. 7 reveals that the prepared acetylcysteine has an ellipsoidal crystal habit with a sphericity degree of 0.875, the crystal habit is complete, and no agglomeration exits.

The particle size of the product obtained in this example was determined by Mastersizer 3000 laser particle size analyzer. The particle size distribution is illustrated in FIG. 8. The particle size distribution is unimodal and has a span of 1.060. The particle size distribution is relatively concentrated, and the mean particle size D[4,3] is 428 µm.

The product obtained in this example has an angle of repose of 26°, which was measured by the fixed funnel method, indicating excellent fluidity.

### Example 4

120.21 g of an acetylcysteine crude product was dissolved in 100.0 mL of water at a preset temperature of 70°C under stirring at a rate of 500 r/min to prepare a saturated solution of acetylcysteine. Then, the solution was cooled to 35°C at rate of 1.5°C/min, and added with 5.11 g of ellipsoidal crystal seeds having a main particle size of 125 µm and a sphericity degree of 0.751, followed by constant-temperature crystal cultivating for 30 min. Thereafter, the obtained solution was cooled to 10°C at a cooling rate of 0.2°C/min, followed by crystal cultivating for 40 min, vacuum filtration, and drying, to obtain 105.47g of acetylcysteine crystals.

The XRD spectrum of the acetylcysteine product prepared in this example is illustrated in FIG. 1. Compared with the two crystal forms in the literature (CrystEngComm, 2013, 15:6498-6505), the peak position of the crystal product obtained by the new crystallization process in Example 4 is consistent with that of crystal form I, indicating that the acetylcysteine product obtained by the new crystallization process is pure crystal form I.

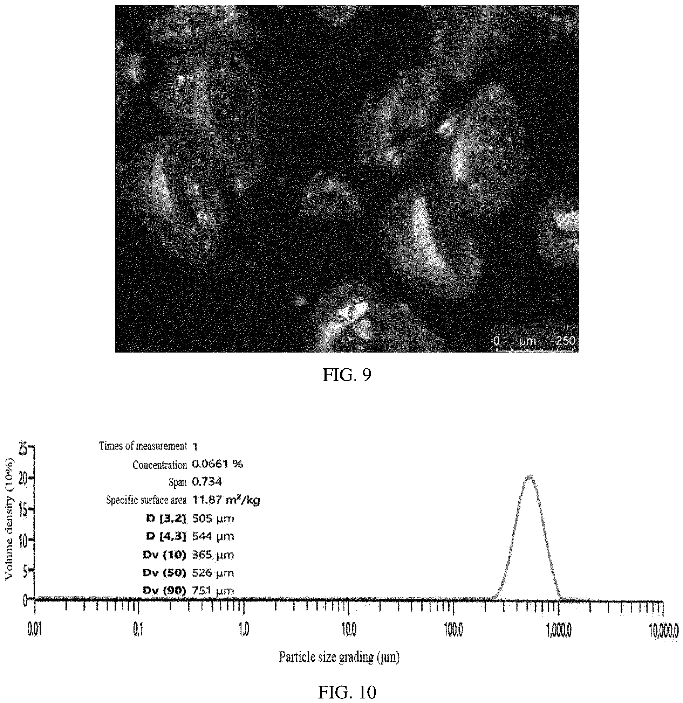

The microscope photograph of the acetylcysteine product prepared in this example is illustrated in FIG. 9. FIG. 9 reveals that the prepared acetylcysteine has an ellipsoidal crystal habit with a sphericity degree of 0.860, the crystal habit is complete, and no agglomeration exits.

The particle size of the product obtained in this example was determined by Mastersizer 3000 laser particle size analyzer. The particle size distribution is illustrated in FIG. 10. The particle size distribution is unimodal and has a span of 0.734. The particle size distribution is relatively concentrated, and the mean particle size D[4,3] is 544 µm.

The product obtained in this example has an angle of repose of 27°, which was measured by the fixed funnel method, indicating excellent fluidity.

### Example 5

210.05g of an acetylcysteine crude product was dissolved in 100.0 mL of water at a preset temperature of 80°C under stirring at a rate of 350 r/min to prepare a saturated solution of acetylcysteine. Then, the solution was cooled to 50°C at a rate of 3.0°C/min, and added with 6.39g of ellipsoidal crystal seeds having a main particle size of 75 µm and a sphericity degree of 0.815, followed by constant-temperature crystal cultivating for 30 min. Thereafter, the obtained solution was cooled to 15°C at a cooling rate of 0.25°C/min, followed by crystal cultivating for 30 min, vacuum filtration, and drying, to obtain 175.28g of acetylcysteine crystals.

The XRD spectrum of the acetylcysteine product prepared in this example is illustrated in FIG. 1. Compared with the two crystal forms in the literature (CrystEngComm, 2013, 15:6498-6505), the peak position of the crystal product obtained by the new crystallization process in Example 5 is consistent with that of crystal form I, indicating that the acetylcysteine product obtained by the new crystallization process is pure crystal form I.

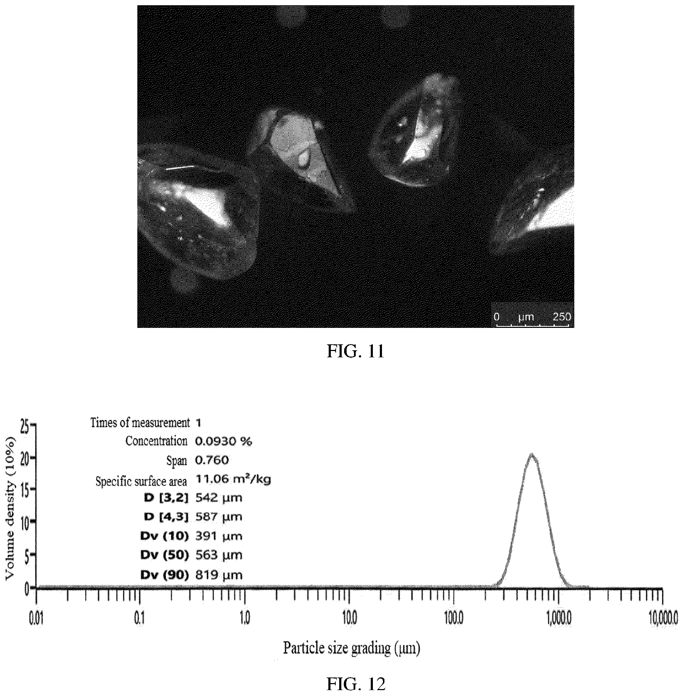

The microscope photograph of the acetylcysteine product prepared in this example is illustrated in FIG. 11. FIG. 11 reveals that the prepared acetylcysteine has an ellipsoidal crystal habit with a sphericity degree of 0.886, the crystal habit is complete, and no agglomeration exits.

The particle size of the product obtained in this example was determined by Mastersizer 3000 laser particle size analyzer. The particle size distribution is illustrated in FIG. 12. The particle size distribution is unimodal and has a span of 0.760. The particle size distribution is relatively concentrated, and the mean particle size D[4,3] is 587 µm.

The product obtained in this example has an angle of repose of 25°, which was measured by the fixed funnel method, indicating excellent fluidity.

Furthermore, the Applicants found that, for other types of amino acids whose solubility in water is close to that of acetylcysteine, the refining method provided by the present disclosure can still obtain relatively high spherical crystal habit and similar particle size distribution. The above experiments were repeated using alanine, arginine, glycine, proline, hydroxyproline, serine, ornithine hydrochloride, arginine hydrochloride, lysine hydrochloride, and histidine hydrochloride, and the obtained results are similar as those in Example 1 to Example 5. As the specific refining process is similar, refining experiments of some amino acids are selected and described in detail as below.

### Example 6: Refining of ornithine hydrochloride

113.03 g of an ornithine hydrochloride crude product was dissolved in 150.0 mL of water at a preset temperature of 55°C under stirring at a rate of 400 r/min to prepare a saturated solution of ornithine hydrochloride. Then, the solution was cooled to 38°C at a rate of 1.5°C/min, and added with 3.40 g of square crystal seeds having a main particle size of 100 µm and a sphericity degree of 0.674, followed by constant-temperature crystal cultivating for 30 min. Thereafter, the obtained solution was cooled to 5°C at a cooling rate of 0.25°C/min, followed by crystal cultivating for 30 min, vacuum filtration, and drying, to obtain 82.45g of ornithine hydrochloride crystals.

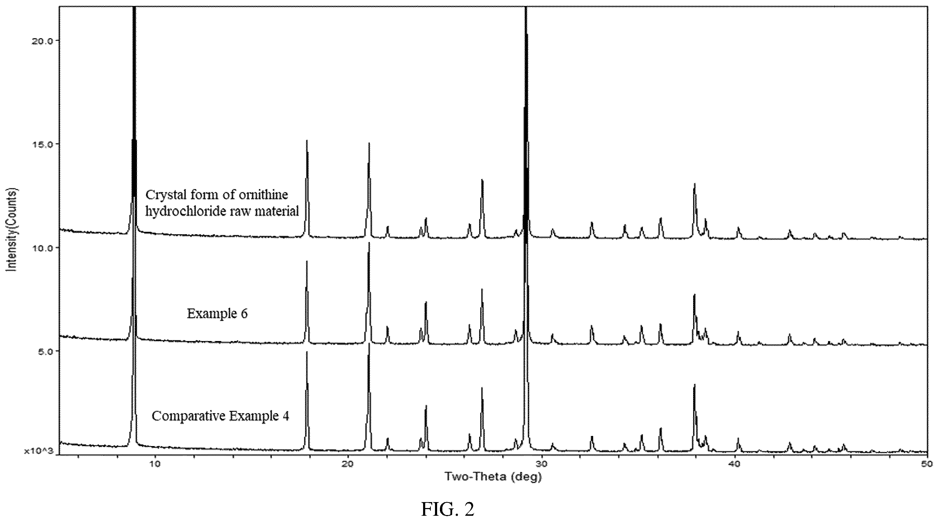

The XRD spectrums of the ornithine hydrochloride product prepared in this example and the raw material are illustrated in FIG. 2. The peak position of the crystal form of the crystal obtained by the new crystallization process in Example 6 is consistent with the peak position of the crystal form of the raw material, indicating that the new crystallization process did not change the crystal form of ornithine hydrochloride.

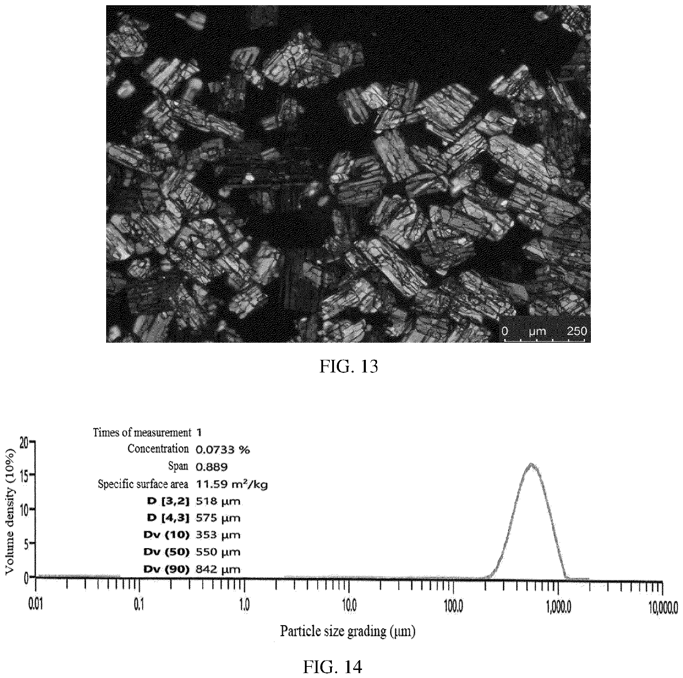

The microscope photographs of the ornithine hydrochloride product prepared in this example and the raw material are illustrated in FIG. 13. FIG. 13 reveals that the prepared ornithine hydrochloride has a square crystal habit with a sphericity degree of 0.805, the crystal habit is complete, and no agglomeration exits.

The particle size of the product obtained in this example was determined by Mastersizer 3000 laser particle size analyzer. The particle size distribution is illustrated in FIG. 14. The particle size distribution is unimodal and has a span of 0.889. The particle size distribution is relatively concentrated, and the mean particle size D[4,3] is 575 µm.

The product obtained in this example has an angle of repose of 30°, which was measured by the fixed funnel method, indicating excellent fluidity.

### Example 7: Refining of glycine

50.05 g of a glycine crude product was dissolved in 100.0 mL of water at a preset temperature of 70°C under stirring at a rate of 450 r/min to prepare a saturated solution of glycine. Then, the solution was cooled to 43°C at a rate of 2°C/min, 1.52 g of ellipsoidal crystal seeds having a main particle size of 100 µm and a sphericity degree of 0.830 was added, followed by constant-temperature crystal cultivating for 30 min. Then, the obtained solution was cooled to 5°C at a rate of 0.5°C/min, followed by crystal cultivating for 30 min, vacuum filtration, and drying, to obtain 39.47 g of glycine crystals.

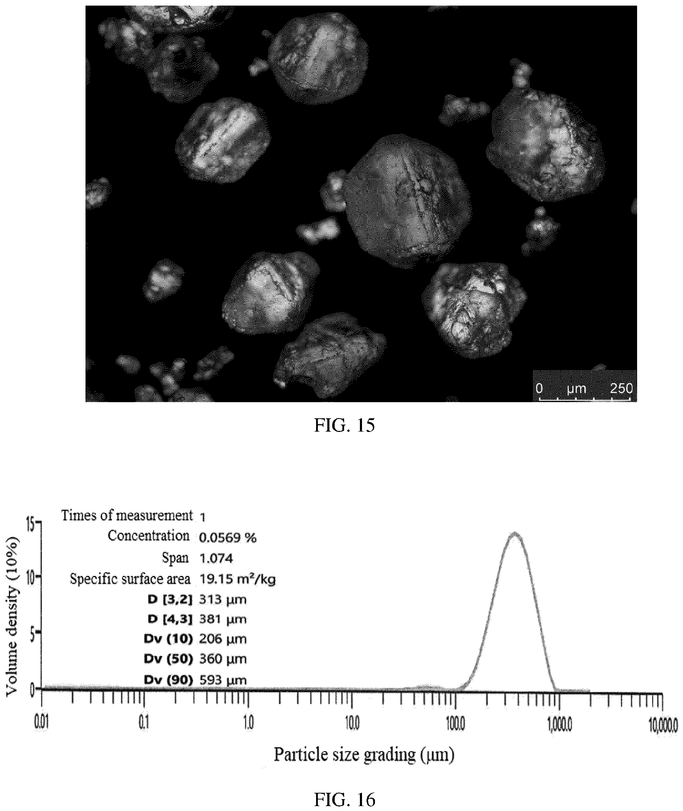

The microscope photograph of the glycine product prepared in this example is illustrated in FIG. 15. FIG. 15 reveals that the prepared glycine has a near-spherical crystal habit with a sphericity degree of 0.895, the habit is complete, and no agglomeration exists.

The particle size of the product obtained in this example was determined by Mastersizer 3000 laser particle size analyzer. The particle size distribution is illustrated in FIG. 16. The particle size distribution is unimodal and has a span of 1.074. The particle size distribution is relatively concentrated, and the mean particle size D[4,3] is 381 µm.

The product obtained in this example has an angle of repose of 24°, which was measured by the fixed funnel method, indicating excellent fluidity.

### Example 8: Refining of alanine

41.06 g of an alanine crude product was dissolved in 100.0 mL of water at a preset temperature of 65°C under stirring at a rate of 350 r/min to prepare a saturated solution of alanine, then the solution was cooled to 34°C at a rate of 2.5°C/min, and added with 1.63 g of ellipsoidal crystal seeds having a main particle size of 125 µm and a sphericity degree of 0.745, followed by constant-temperature crystal cultivating for 30 min. Then, the obtained solution was cooled to 5°C at a cooling rate of 0.3°C/min, followed by crystal cultivating for 30 min, vacuum filtration, and drying, to obtain 31.47 g of glycine crystals.

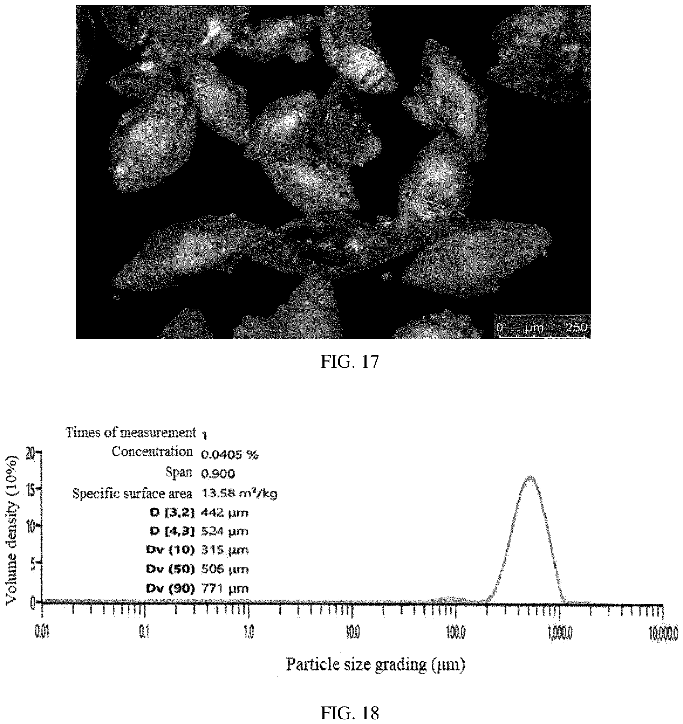

The microscope photograph of the glycine product prepared in this example is illustrated in FIG. 17. FIG. 17 reveals that the prepared glycine has an ellipsoidal crystal habit with a sphericity degree of 0.824, the crystal habit is complete, and no agglomeration exits.

The particle size of the product obtained in this example was determined by Mastersizer 3000 laser particle size analyzer. The particle size distribution is illustrated in FIG. 18. The particle size distribution is unimodal and has a span of 0.900. The particle size distribution is relatively concentrated, and the mean particle size D[4,3] is 524 µm.

The product obtained in this example has an angle of repose of 28°, which was measured by the fixed funnel method, indicating excellent fluidity.

### Comparative Example 1: without two-steps gradient cooling

100.04 g of an acetylcysteine crude product was dissolved in 100.0 mL of water at a preset temperature of 60°C under stirring at a rate of 400 r/min to prepare a saturated solution of acetylcysteine, and then 3.5g of ellipsoidal crystal seeds having a main particle size of 100 µm and a sphericity degree of 0.815 was added. The solution was cooled rapidly to 5°C at a rate of 2.0°C/min, followed by crystal cultivating, vacuum filtration, and drying, to obtain 75.98 g of acetylcysteine crystals. The obtained crystal was a slender and pointed crystal habit, and the phenomenon of material attachment occurred.

The XRD pattern of the acetylcysteine product prepared by this comparative example is illustrated in FIG. 1. Compared with the two crystal forms in the literature (CrystEngComm, 2013, 15:6498-6505), the peak position of the obtained product is consistent with that of crystal form I, indicating that the acetylcysteine product obtained by Comparative Example 1 is crystal form I.

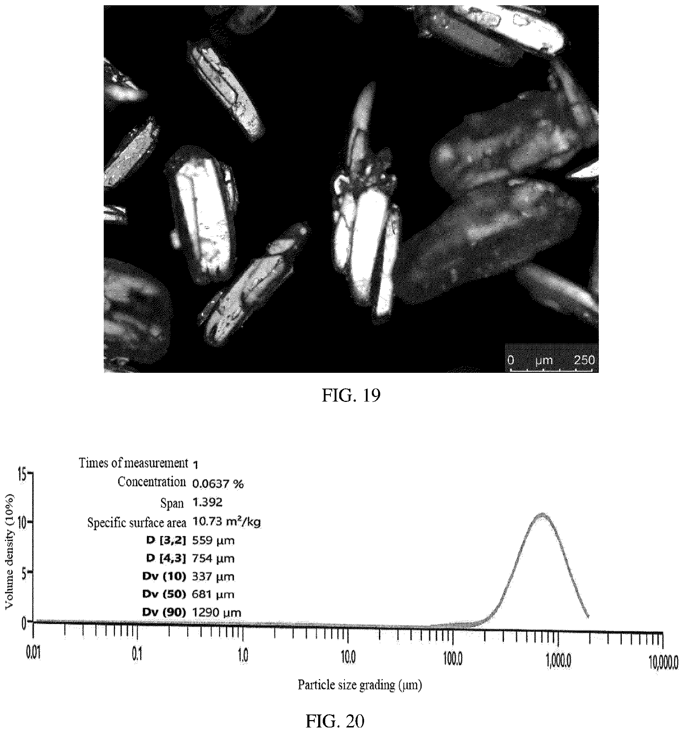

The microscope photograph of the acetylcysteine product prepared in this comparative example is illustrated in FIG. 19. FIG. 19 reveals that the acetylcysteine in Comparative Example 1 has problems such as uneven particle size distribution, agglomeration, and fragmentation. It can be observed under a polarizing microscope that the acetylcysteine crystal habit obtained by Comparative Example 1 is slender and pointed and is prone to be broken during the crystallization process, the sphericity degree is 0.510, which is excessively small and may result in poor fluidity and proneness to carry static electricity.

The particle size of the product obtained by this comparative example is determined by the Mastersizer 3000 Marvin particle size analyzer. The particle size distribution is illustrated in FIG. 20. The particle size distribution is too wide and has a span of 1.392, and the mean particle size D[4,3] is 754 µm.

The product obtained by this comparative example has an angle of repose of 48°, measured by the fixed funnel method, indicating poor liquidity.

### Comparative Example 2: crystal cultivating using ethanol as solvent

80.55 g of an acetylcysteine crude product was dissolved in 100.0 mL of ethanol solvent at a preset temperature of 50°C under stirring at a rate of 350 r/min to prepare a saturated solution of acetylcysteine. Then, the solution was cooled to 27°C at a rate of 1.0°C/min, and added with 2.56 g of crystal seeds having a main particle size of 50 µm and a sphericity degree of 0.796, followed by constant-temperature crystal cultivating for 30 min. Then, the obtained solution was cooled to 10°C at a cooling rate of 0.1°C/min, followed by crystal cultivating for 30 min, vacuum filtration, and drying, to obtain 48.45 g of acetylcysteine crystal, with a low yield.

The XRD pattern of the acetylcysteine product prepared by this comparative example is illustrated in FIG. 1. Compared with the two crystal forms in the literature (CrystEngComm, 2013, 15:6498-6505), the peak position of the obtained product is consistent with that of crystal form I, indicating that the acetylcysteine product obtained by this comparative example is crystal form I.

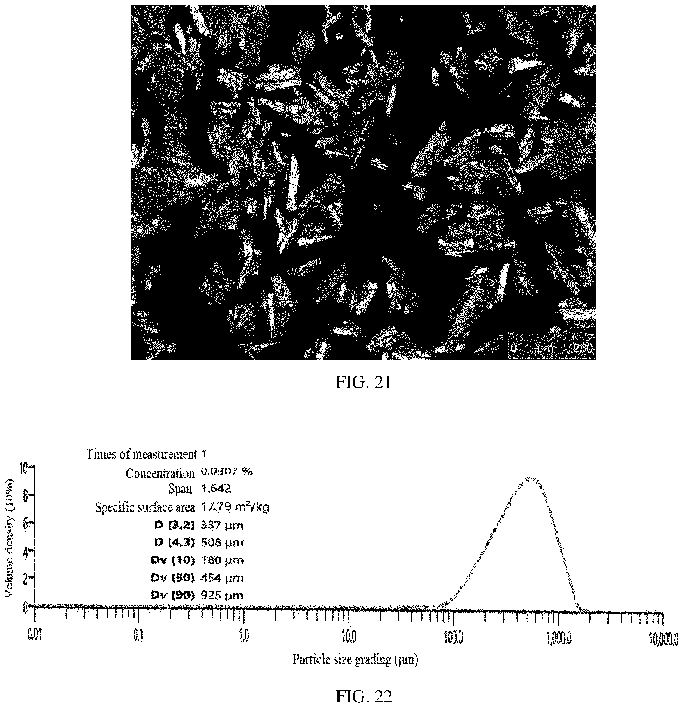

The microscope photograph of the acetylcysteine product prepared in Comparative Example 2 is illustrated in FIG. 21. FIG. 21 reveals that the obtained product has an uneven particle size distribution and is prone to be broken. It can be observed under the polarizing microscope that the acetylcysteine crystal habit obtained in Comparative Example 2 is long rod shaped and is prone to be broken during the crystallization process, and the sphericity degree is 0.435, which is excessively low and may result in poor mobility and proneness to carry static electricity.

The particle size of the product obtained by this comparative example was determined by Mastersizer 3000 Malvern particle size analyzer. The particle size distribution is illustrated in FIG. 22. The particle size distribution is too wide and has a span of 1.642, and the mean particle size D[4,3] is 508 µm.

The product obtained in this comparative example has an angle of repose of 50°, which was measured by the fixed funnel method, indicating poor fluidity.

The Applicants conducted further experiments using propanol, isopropanol, and other organic solvents, and the obtained results are similar as this comparative example, which will not be repeated herein.

### Comparative Example 3: crystal cultivating using long pointed crystal seeds

210.00 g of an acetylcysteine crude product was dissolved in 100.0 mL of water at a temperature of 80°C under stirring at a rate of 350 r/min. Then, the solution was cooled to 53°C at a rate of 3.0°C/min, and added with 6.05 g of long pointed crystal seeds having a main particle size of 75 µm and a sphericity degree of 0.413, followed by constant-temperature crystal cultivating for 30 min. Thereafter, the obtained solution was cooled to 15°C at a rate of 0.25°C/min, followed by crystal cultivating for 30 min, vacuum filtration, and drying, to obtain 158.35g of acetylcysteine crystals.

The XRD pattern of the acetylcysteine product prepared by this comparative example is illustrated in FIG. 1. Compared with the two crystal forms in the literature (CrystEngComm, 2013, 15:6498-6505), the peak position of the obtained product is consistent with that of crystal form I, indicating that acetylcysteine product obtained in this comparative example is crystal form I.

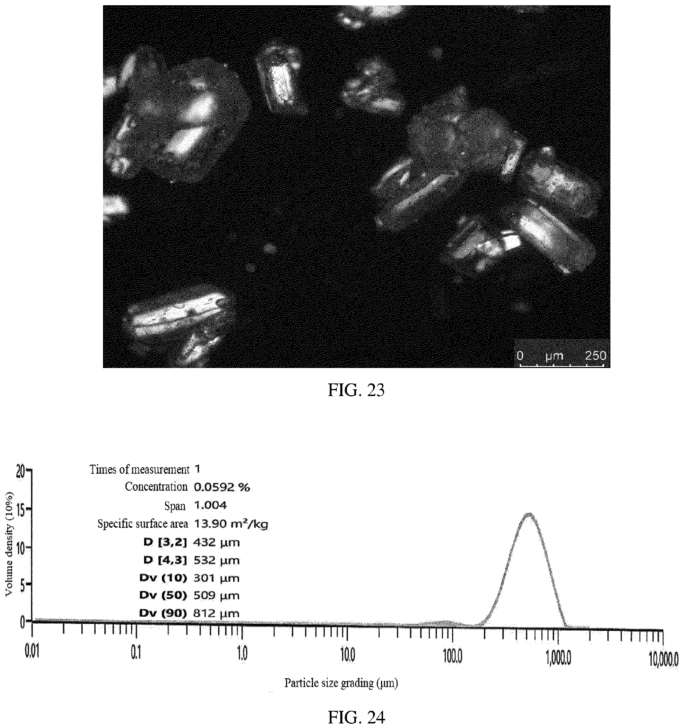

The microscope photograph of the acetylcysteine product prepared in Comparative Example 3 is illustrated in FIG. 23. The polarized microscope photograph reveals that the acetylcysteine crystal habit is of the shape of cuboids and is prone to be broken, and the sphericity degree is 0.655, which is excessively small and may result in poor fluidity.

The particle size of the product obtained by this comparative example was determined by Mastersizer 3000 Malvern particle size analyzer. The particle size distribution is illustrated in FIG. 24. The particle size distribution is uneven and has a span of 1.004, and the mean particle size D[4,3] is 532 µm.

The product obtained in this comparative example has an angle of repose of 46°, which was measured by the fixed funnel method, indicating poor liquidity.

### Comparative Example 4: crystal cultivating without adding crystal seeds

112.00 g of an ornithine hydrochloride crude product was dissolved in 150.0 mL of water at a temperature of 60°C under stirring at a rate of 400 r/min. Then, the solution was directly cooled to 5°C at 1.5°C/min, followed by crystal cultivating for 30 min, vacuum filtration, and drying, to obtain 75.34 g of ornithine hydrochloride crystals.

The XRD patterns of the obtained ornithine hydrochloride product and the raw material in this example are illustrated in FIG. 2. The peak position of the crystal form of the crystals obtained in Comparative Example 4 is consistent with the peak position of the crystal form of the raw material, indicating that the crystal cultivating without adding crystal seeds did not change the crystal form of ornithine hydrochloride.

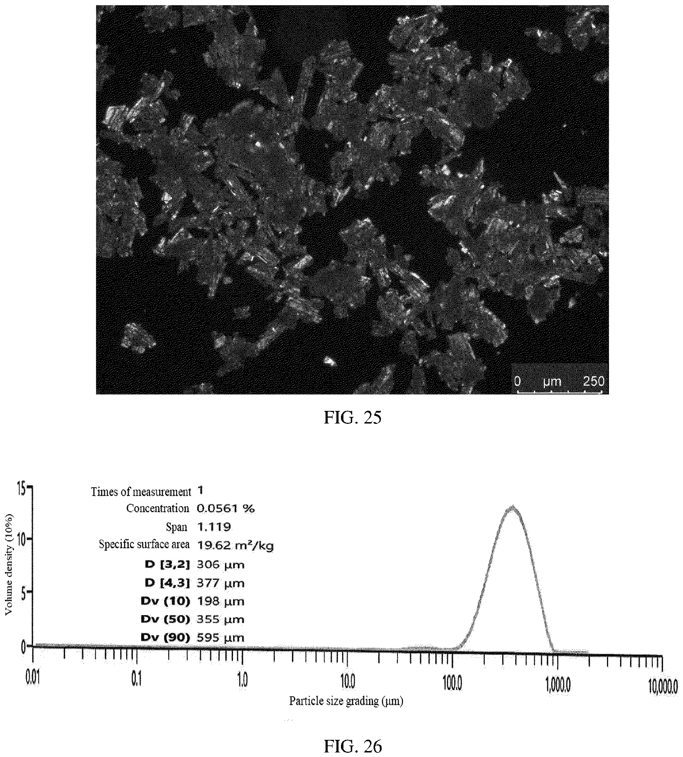

The microscope photograph of the ornithine hydrochloride product prepared in this comparative example is illustrated in FIG. 25. FIG. 25 reveals that the crystal habit of the prepared ornithine hydrochloride is fine needle shaped and is extremely prone to be broken, extremely serious agglomeration occurred, and the sphericity degree is 0.346, which is excessively small.

The particle size of the product obtained in this example was determined by Mastersizer 3000 laser particle size analyzer. The particle size distribution is illustrated in FIG. 26. The particle size distribution is uneven and has a span of 1.119, and the mean particle size D[4,3] is 377 µm.

The product obtained in this comparative example has an angle of repose of 54°, which was measured with the fixed funnel method, indicating poor liquidity.

The experimental parameters and crystal parameters of all the examples of the present disclosure and comparative examples are illustrated in Table 2.

**Table 2: Experimental parameters and crystal parameters of examples and comparative examples**

| **Example No.** | **Raw material** | **Solvent** | **Initial concentration g/100mL** | **Stage cooling rate °C/min (first/second programmed cooling)** | **Stirring rate rpm** | **End temperature °C** | **Sphericity degree** | **Mean particle size µm** | **Span** |
|---|---|---|---|---|---|---|---|---|---|
| Example 1 | Acetylcysteine crude product | Pure water | 200.00 | 1.0/0.3 | 300 | 10 | 0.865 | 273 | 0.914 |
| Example 2 | | Pure water | 150.00 | 1.25/0.5 | 350 | 5 | 0.881 | 360 | 1.021 |
| Example 3 | | Pure water | 100.00 | 2.0/0.8 | 400 | 5 | 0.875 | 428 | 1.060 |
| Example 4 | | Pure water | 120.21 | 1.5/0.2 | 500 | 10 | 0.860 | 544 | 0.734 |
| Example 5 | | Pure water | 210.05 | 3.0/0.25 | 350 | 15 | 0.886 | 587 | 0.760 |
| Example 6 | Ornithine hydrochloride crude product | Pure water | 75.35 | 1.5/0.25 | 400 | 5 | 0.805 | 575 | 0.889 |
| Example 7 | Glycine | Pure water | 50.05 | 2.0/0.5 | 450 | 5 | 0.895 | 381 | 1.074 |
| Example 8 | Alanine | Pure water | 41.06 | 2.5/0.3 | 350 | 5 | 0.824 | 524 | 0.900 |
| Comparative Example 1 | Acetylcysteine crude product | Pure water | 100.04 | 2.0 | 400 | 5 | 0.510 | 754 | 1.392 |
| Comparative Example 2 | | ethanol | 80.55 | 1.0/0.1 | 350 | 10 | 0.435 | 508 | 1.642 |
| Comparative Example 3 | | Pure water | 210.00 | 3.0/0.25 | 350 | 15 | 0.655 | 532 | 1.004 |
| Comparative Example 4 | Ornithine hydrochloride crude product | Pure water | 74.67 | 1.5 | 400 | 5 | 0.346 | 377 | 1.119 |

Further, the Applicants measured the fluidity of the refined products prepared in the examples and comparative examples and the fluidity of the crude products used, and the results thereof are illustrated in Table 3. It can be seen from the results that the amino acid or derivatives thereof prepared by the present disclosure have excellent fluidity performance and high crystal stability, and thus they are suitable for direct tableting, thereby effectively improving the quality of tablet products.

**Table 3: Fluidity test of refined products prepared by examples and comparative examples and crude products used**

| Sample No. | Angle of repose ° | Fluidity |
|---|---|---|
| Acetylcysteine crude product | 52 | Poor |
| Example 1 | 28 | Excellent |
| Example 2 | 26 | Excellent |
| Example 3 | 26 | Excellent |
| Example 4 | 27 | Excellent |
| Example 5 | 25 | Excellent |
| Comparative Example 1 | 48 | Poor |
| Comparative Example 2 | 50 | Poor |
| Comparative Example 3 | 46 | Poor |
| Ornithine hydrochloride crude product | 65 | Poor |
| Example 6 | 30 | Excellent |
| Comparative Example 4 | 54 | Poor |
| Glycine crude product | 52 | Poor |
| Example 7 | 24 | Excellent |
| Alanine crude product | 55 | Poor |
| Example 8 | 28 | Excellent |

When the angle of repose is smaller than or equal to 30°, the fluidity is excellent; when the angle of repose is in the range from 30° to 45°, the fluidity is good; and when the angle of repose is greater than 45°, the liquidity is poor.

To sum up, with the method of the present disclosure, the crystal form of the product is not changed, and the obtained products have a pure crystal form, which does not affect the bioavailability in vivo. The crystal product has a complete crystal habit and a high sphericity degree, the crystal particle size distribution is unimodal and concentrated, having a relatively small span. Moreover, products with different particle size distributions can be obtained through the control of the crystallization process to meet the requirements of different particle sizes.

In the description of this specification, description with reference to the terms "an embodiment", "some embodiments", "examples", "specific examples", or "some examples" means that the specific features, structures, materials, or characteristics described in combination with this embodiment or example are included in at least one embodiment or example of the present disclosure. In this specification, the illustrative expression of the above terms does not necessarily refer to the same embodiments or examples. Furthermore, the specific features, structures, materials, or characteristics described may be combined in an appropriate manner in any one or more embodiments or examples. In addition, those skilled in the art can combine different embodiments or examples described in this specification and the features of different embodiments or examples, unless they are contradictory with each other.

The embodiments of the present disclosure illustrated and described above are merely exemplary and cannot be understood as a limitation of the present disclosure. Those skilled in the art can make change, modification, replacement, and transformation to the above embodiments within the scope of the present disclosure.

## Claims

1. A refining method of an amino acid or derivatives thereof, the method comprising:
step 1 of preparing a saturated aqueous solution of the amino acid or derivatives thereof by using a crude product of the amino acid or derivatives thereof at a preset temperature;
step 2 of cooling, in a first programmed cooling, the saturated aqueous solution to a metastable zone, adding crystal seeds having a sphericity degree not less than 0.6, and performing constant-temperature crystal cultivating to obtain a crystal slurry; and
step 3 of cooling, in a second programmed cooling, the crystal slurry to a second temperature with a cooling rate not higher than 1.0°C/min, and performing constant-temperature crystal cultivating to obtain the amino acid or derivatives thereof.

2. The method according to claim 1, wherein the preset temperature ranges from 40°C to 80°C, preferably from 50°C to 80°C.

3. The method according to claim 1, wherein the second temperature ranges from 0°C to 25°C, preferably from 5°C to 15°C.

4. The method according to claim 1, wherein in step 2, said cooling, in the first programmed cooling, the saturated aqueous solution to the metastable zone is carried out under stirring, optionally a speed of said stirring being 100 r/min to 600 r/min, and preferably the speed of said stirring being 300 r/min to 500 r/min.

5. The method according to claim 1, wherein the cooling rate of the second programmed cooling ranges from 0.2°C/min to 1.0°C/min.

6. The method according to claim 1, wherein the sphericity degree of the crystal seeds preferably ranges from 0.6 to 0.9; the crystal seeds are added in an amount of 1% to 15% by mass, based on the saturated aqueous solution; and the crystal seeds have a main particle size ranging from 10 µm to 200 µm;
preferably, the crystal seeds are added in an amount of 2% to 6% by mass, and the crystal seeds have the main particle size ranging from 50 µm to 125 µm.

7. The method according to claim 1, wherein the crude product of the amino acid or derivatives thereof has a crystal habit sphericity degree not higher than 0.5.

8. The method according to claim 1, wherein the constant-temperature crystal cultivating is performed for 5 min to 60 min, preferably for 20 min to 40 min.

9. The method according to claim 1, further comprising, subsequent to said cooling the crystal slurry in the second programmed cooling and said performing the constant-temperature crystal cultivating:
performing centrifugal filtration and drying on the product,
optionally, a rotating speed of the centrifugal filtration ranges from 1,000 rpm to 5,000 rpm, preferably from 3,000 rpm to 4,500 rpm;
optionally, the drying is selected from at least one of atmospheric drying at 75 °C to 80°C, vacuum drying, or rotary evaporation drying.

10. The method according to claim 1, wherein the metastable zone is between 25°C and 50°C, and preferably between 27°C and 45°C.

11. The method according to claim 1, wherein the amino acid or derivatives thereof prepared by the method has a crystal habit sphericity degree not less than 0.8.

12. The method according to claim 1, wherein the amino acid or derivative thereof prepared by the method has a span of particle size distribution not higher than 1.1.

13. The refining method of the amino acid or derivatives thereof according to any one of claims 1 to 12, wherein:
the amino acid or derivatives thereof comprises at least one of acetylcysteine, alanine, arginine, glycine, proline, hydroxyproline, serine, ornithine hydrochloride, arginine hydrochloride, lysine hydrochloride, or histidine hydrochloride,
preferably, the amino acid or derivatives thereof comprises acetylcysteine, ornithine hydrochloride, glycine, or alanine; and
more preferably, the amino acid is acetylcysteine.

14. An amino acid or derivatives thereof, having a crystal habit sphericity degree not less than 0.8.

15. The amino acid or derivatives thereof according to claim 14, wherein the amino acid or derivatives thereof has a span of particle size distribution not higher than 1.1.

16. The amino acid or derivatives thereof according to claim 14, wherein:
the amino acid or derivatives thereof comprises at least one of acetylcysteine, alanine, arginine, glycine, proline, hydroxyproline, serine, ornithine hydrochloride, arginine hydrochloride, lysine hydrochloride, or histidine hydrochloride;
preferably, the amino acid or derivatives thereof comprises acetylcysteine, ornithine hydrochloride, glycine, or alanine; and
more preferably, the amino acid is acetylcysteine.

17. A refining method of acetylcysteine, comprising:
preparing a saturated aqueous solution of acetylcysteine by using an acetylcysteine crude product at 50°C to 80°C;
cooling the saturated aqueous solution to a metastable zone between 27°C and 45°C at a rate of 1°C/min to 3.0°C/min, adding crystal seeds having a sphericity degree not less than 0.6, and performing constant-temperature crystal cultivating to obtain a crystal slurry; and
cooling the crystal slurry to 5°C to 15°C at a rate of 0.2°C/min to 0.8°C/min, and performing constant-temperature crystal cultivating to obtain refined acetylcysteine.
